# EUROPEAN PATENT APPLICATION

(11) **EP 1 518 794 A1**
(43) Date of publication of application: **30.03.2005**
(21) Application number: 04022252.3
(22) Date of filing: 17.09.2004
(51) Int. Cl.: B65D 75/58, B65D 75/32, A61L 9/12

(54) **Container for dispensing volatile substances**

(30) Priority: 19.09.2003 IT BO20030546
(71) Applicant: FALP S.r.l., 40060 Passo Segni Baricella (Bologna) (IT)
(72) Inventor: Falchieri, Roberto, 40060 Passo Segni di Baricella (BO) (IT)
(74) Representative: Modiano, Guido, Dr.-Ing.

(57) **Abstract**

A container for dispensing volatile substances, of the type provided with a box-like shell (2) made of impermeable material, having an outer perimetric edge (3) along which the outer edge (4) of a semipermeable film (5) is heat-sealed, the film being associated with a film (6) made of metallic material that can be removed before use, the lower portion (7) of the box-like shell (2) being considerably lower and having longitudinal ribs (9a, 9b) that form a plurality of flattened longitudinal evaporation regions (10a, 10b, 10c), the upper edge of the box-like shell being provided with a channel (11) which, together with the semipermeable film (5), forms a duct (12) for the inflow of the compensating air that replaces the volume of the volatilized substance in order to avoid the collapse of the container.

## Description

The present invention relates to a container for dispensing volatile substances, such as deodorants or the like.

In the particular field of the dispensing of volatile substances, such as for example room deodorants, dispensers are known which comprise a box-like shell made of impermeable material, with an outer perimetric edge along which the outer edge of a semipermeable film is heat-sealed, said film being adapted to allow the fragrance of the contained liquid to flow out without allowing said liquid to flow out; a film made of metallic material is associated with the semipermeable film and is adapted to make the surface of the film impermeable before use.

After removing the film made of metallic material, the semipermeable membrane allows the volatile components of the contents to pass through it outward and retains the liquid components thereof.

Currently, known disposable containers have drawbacks due to the fact that, since their cross-section is substantially constant over their entire height, as the content of liquid decreases, the surface of contact of the liquid with the membrane made of semipermeable material also decreases, and therefore the dispensing of the contents decreases.

Moreover, in known containers it is practically impossible to interrupt the emission of the deodorant after the metallic film that protects the semipermeable membrane has been eliminated.

The aim of the present invention is to obviate the above-cited drawbacks and to meet the mentioned requirements, by providing a container for dispensing volatile substances that maintains, throughout its use, a large contents emission surface and has a sort of valve that allows to interrupt at any time the dispensing of the volatile substance by turning upside down the container so that the duct is directed downward, without the contained liquid dripping out.

Within this aim, an object of the present invention is to provide a container for dispensing volatile substances that has a simple structure, is relatively easy to provide in practice, safe in use, effective in operation, and has a relatively low cost.

This aim and this and other objects that will become better apparent hereinafter are achieved by the present container for dispensing volatile substances, of the type provided with a box-like shell made of impermeable material, having an outer perimetric edge along which the outer edge of a semipermeable film is heat-sealed, said film being associated with a film made of metallic material that can be removed before use, characterized in that the lower portion of the box-like shell is considerably lower than the upper portion and has longitudinal ribs that form a plurality of flattened longitudinal evaporation regions, and in that in the upper edge of the box-like shell there is a channel which, together with the semipermeable film, forms a duct for the inflow of the compensating air that replaces the volume of the volatilized substance in order to avoid the collapse of the container.

Further characteristics and advantages of the invention will become better apparent from the following detailed description of a preferred but not exclusive embodiment of a container for dispensing volatile substances according to the invention, illustrated by way of non-limiting example in the accompanying drawings, wherein:

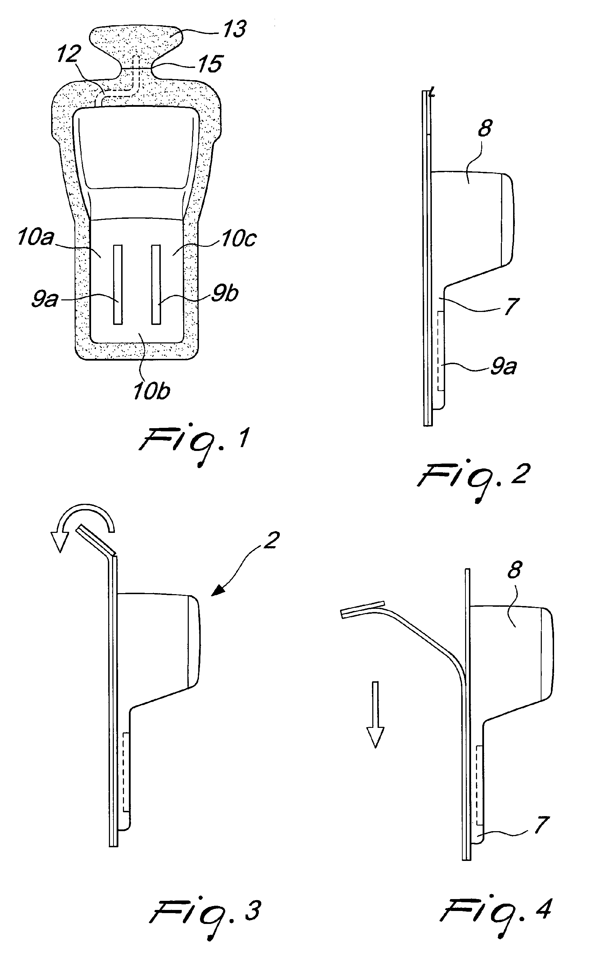

Figure 1 is a front view of a container for dispensing volatile substances according to the invention, in the intact condition for transport and sale;
Figure 2 is a side view of the container, in the intact condition;
Figure 3 is a side view of the container in an initial step of the elimination of the protective film;

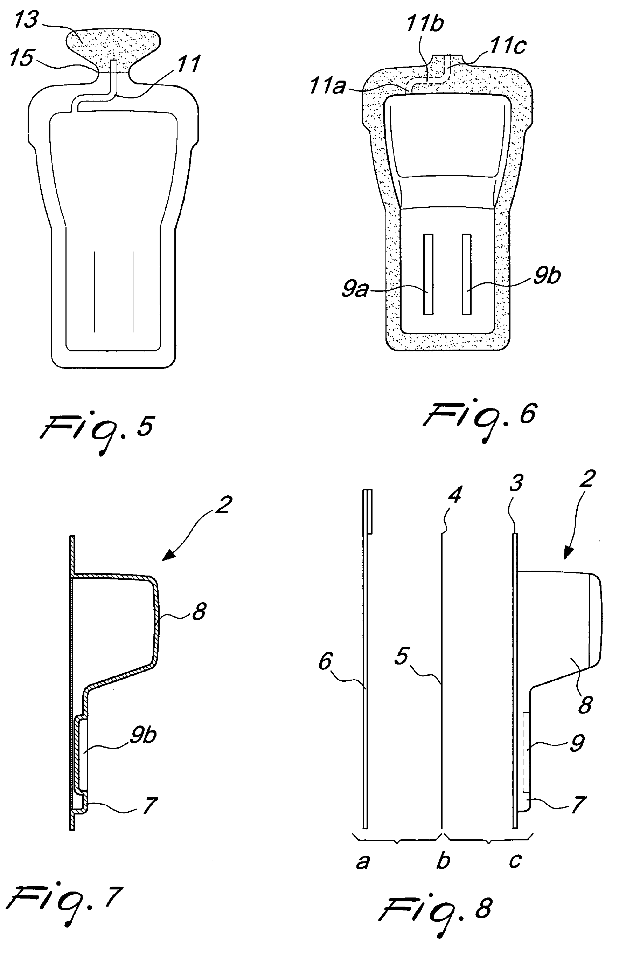

Figure 4 is a side view of the container in a subsequent step of the elimination of the protective film;
Figure 5 is a front view of the protective film after removal;
Figure 6 is a front view of the container for dispensing volatile substances in the active configuration;
Figure 7 is a side view of the container for dispensing volatile substances in the active configuration;
Figure 8 is an exploded side view, in which the reference letter *a* designates the protective part for transport, which is removed before use, the reference letter b designates the semipermeable film, and the reference letter c designates the box-like shell.

With reference to the figures, the reference numeral 1 generally designates a container for dispensing volatile substances according to the invention.

The container 1 is of the type constituted by a box-like shell 2 made of impermeable material, with an outer perimetric edge 3 along which the outer edge 4 of a semipermeable film 5 is heat-sealed; said film is associated with a film 6 made of a metallic material, which can be removed before use.

Advantageously, the shell 2 is provided by molding a material such as clear plastics, particularly A-PET/PE, the semipermeable film is made of a material such as polyester, and the film 6 is made of a metallic material of the type known as aluminized film.

The lower portion 7 of the box-like shell 2 is considerably lower than the upper part 8 and has, in the illustrated embodiment, two longitudinal ribs 9a and 9b, which form a plurality of flattened longitudinal evaporation regions, in the particular case three regions 10a, 10b and 10c.

A channel 11 is formed in the upper edge of the box-like shell and forms, together with the semipermeable film 5, a narrow duct 12 for the inflow of the compensating air that gradually replaces the volume of the volatilized substance.

The semipermeable film 5 and the film 6 made of metallic material are extended by means of a tab 13 that can be removed by tearing, is shaped substantially like an isosceles trapezoid with rounded comers, and is rigidly coupled to a corresponding tab 14 that extends the edge of the box-like shell, a shear line 15 being provided at the narrower portion at the base of the tab of the shell in order to separate manually the tab from the shell and remove the film made of metallic material from the evaporation surface of the film.

It should be noted that the narrow duct 11 advantageously forms a bend, which is constituted by two rising portions 11 a and 11b that are mutually spaced by a horizontal portion 11c, the ends of said portions being mutually connected by curved sections.

Conveniently, an elbow is formed which reaches below the base of the shell, with two vertical portions that reach the base, where they join the horizontal portion: this forms a full-height siphon, which gives assurance that the container, if turned upside down so that the open end of the duct faces downward, does not drip.

The longitudinal ribs 9a and 9b are heat-sealed to the semipermeable film 5 in order to prevent the semipermeable film from bulging.

The operation of the container according to the invention is as follows: by removing the removable tab, first of all the duct 11 is opened toward the environment, so that air for compensating the volume of liquid that has evaporated into the environment can enter through the duct 11: since the bulkiest part of the container is open toward the duct and is arranged in an upper region, the container according to the invention is less sensitive to the decrease over time of the substance to be evaporated, since the surface for evaporation and exchange with the environment is constituted, almost up to the end of the product, at least by the surface of the film of the evaporation regions, which is slightly less than half of the entire surface.

It has thus been shown that the invention achieves the intended aim and object.

The invention thus conceived is susceptible of numerous modifications and variations, all of which are within the scope of the appended claims.

All the details may further be replaced with other technically equivalent ones.

In the described embodiments, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

The disclosures in Italian Patent Application No. BO2003A000546, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A container for dispensing volatile substances, of the type provided with a box-like shell (2) made of impermeable material, having an outer perimetric edge (3) along which the outer edge (4) of a semipermeable film (5) is heat-sealed, said film being associated with a film (6) made of metallic material that can be removed before use, **characterized in that** the lower portion (7) of the box-like shell (2) is considerably lower than the upper portion and has longitudinal ribs (9a, 9b) that form a plurality of flattened longitudinal evaporation regions (10a, 10b, 10c), and **in that** in the upper edge of the box-like shell there is a channel (11) which, together with the semipermeable film (5), forms a duct (12) for the inflow of the compensating air that replaces the volume of the volatilized substance in order to avoid the collapse of the container.

2. The container according to claim 1, **characterized in that** the upper edge of the semipermeable film (5) and of the film (6) made of metallic material are extended by means of a tab (13) that can be removed by tearing and is coupled to a corresponding tab (14) that extends the edge (3) of the box-like shell (2), a shear line (15) being provided at the narrower region at the base of said tab of the shell (14) in order to separate manually the tab (13) from the shell (2) and remove the film (6) made of metallic material from the evaporation surface.

3. The container according to one or more of the preceding claims, **characterized in that** said duct (12) forms a bend that is suitable to prevent dripping in case of accidental overturning.

4. The container according to claim 3, **characterized in that** said bend forms an elbow that reaches below the base of the shell (2).

5. The container according to one or more of the preceding claims, **characterized in that** said longitudinal ribs (9a, 9b) are heat-sealed to the semipermeable film (5) in order to prevent the film (5) from bulging when the container is upside down in the active configuration.
